(19) Europäisches Patentamt
European Patent Office
Office européen des brevets

(11) Publication number: 0 254 229
A1

(12) **EUROPEAN PATENT APPLICATION**

(21) Application number: 87110406.3

(22) Date of filing: 17.07.87

(51) Int. Cl.³: C 07 C 85/04
C 07 C 87/14

(30) Priority: 18.07.86 JP 167899/86

(43) Date of publication of application: 27.01.88 Bulletin 88/4

(84) Designated Contracting States: BE CH DE FR GB IT LI NL

(71) Applicant: MITSUI TOATSU CHEMICALS INC.
No. 2-5, Kasumigaseki 3-chome
Chiyoda-Ku Tokyo 100(JP)

(72) Inventor: Nagata, Teruyuki
154, Shiragane-machi
Omuta-shi Fukuoka(JP)

(72) Inventor: Kajimoto, Nobuyuki
300, Hirabaru-machi
Omuta-shi Fukuoka(JP)

(72) Inventor: Watanabe, Katsuji
86, Hachihon-machi
Omuta-shi Fukuoka(JP)

(74) Representative: Strehl, Schübel-Hopf, Groening, Schulz
Widenmayerstrasse 17 Postfach 22 03 45
D-8000 München 22(DE)

(54) **Process for producing N,N'-dialkylalkanediamines.**

(57) A process for producing of N,N'-dialkylalkanediamines represented by the formulae (I), (II) or (III),

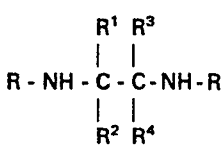
(I)

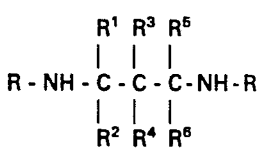
(II)

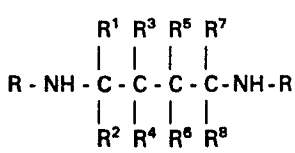
(III)

wherein R is a lower alkyl group and $R^1$ to $R^8$ are each hydrogen atom or a lower alkyl group, which comprises reacting the corresponding dichloroalkanes represented by Formulae (IV), (V) or (VI),

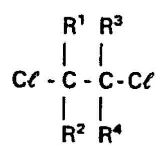
(IV)

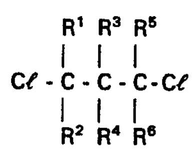
(V)

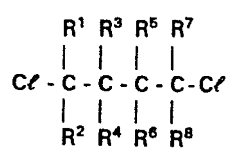
(VI)

wherein $R^1$ to $R^8$ are as defined above with a lower alkyl primary amine represented by Formula (VII),

$$R\text{-}NH_2 \qquad (VII)$$

wherein R is as defined above substantially in the absence of water.

EP 0 254 229 A1

Croydon Printing Company Ltd.

TITLE OF THE INVENTION

Process for Producing N,N'-dialkylalkanediamines

BACKGROUND OF THE INVENTION

This invention relates to a process for producing N,N'-dialkylalkanediamines represented by Formulae (I), (II) or (III),

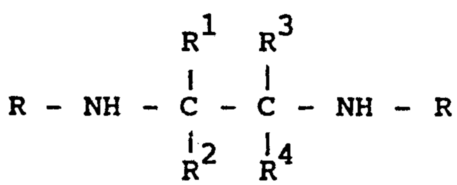 (I)

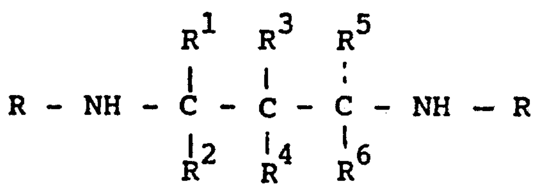 (II)

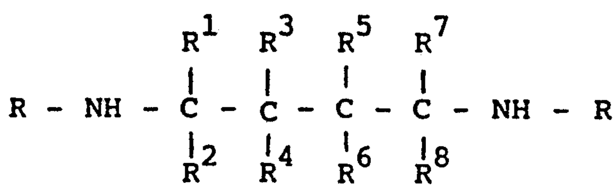 (III)

wherein R is a lower alkyl group and $R^1$ to $R^8$ each are a hydrogen atom or a lower alkyl group.

These N,N'-dialkylalkanediamines are compounds useful as intermediates in the organic synthesis chemistry and particularly, as intermediates of N,N'-dialkyl cyclic ureas used as a non-protonic polar solvent.

There have been provided processes for the production of these N,N'-dialkylalkanediamines, for example, production

of N,N'-dimethyl-1,2-ethanediamine by reaction of dibromoalkane with alkylamine [Journal of Chemical Society, 314, (1947)] and production of. N,N'-dimethyl-1,3-propanediamine by reaction of dibromoalkane with alkylamine [Journal of Chemical Society, 168, (1942)].

Further, a process for the production of N,N'-dialkylalkanediamines by reaction of dichloroalkanes with lower alkyl primary amines is known in, for example, Japanese Patent Kokai Koho No. 57-120570, according to Examples of which it is disclosed that in the preparation of 1,3-dimethyl-2-imidazolidinone by reacting 1,2-dichloroethane with liquefied methylamine and $CO_2$ gas in the presence of water under elevated pressure N,N'-dimethyl-1,2-ethanediamine is obtained as the intermediate in the same reaction vessel. However, since the N,N'-dimethyl-1,2-ethanediamine is not isolated or analysed, its yield is unclear.

Referring to the process for production of N,N'-dialkylalkanediamines by reaction of dibromoalkanes with alkylamines as mentioned above, expensive bromo compounds are used as starting materials and therefore, there is room for further improvement in the industrial process for production.

The inventors had considered the process for production of N,N'-dialkylalkanediamines by reaction of dichloroalkanes with lower alkyl primary amines to be most convenient industrially and conducted the reaction of 1,2-dichloroethane with

an excess of methylamine in the form of a 40 % aqueous solution of methylamine available easily, however, as a result the yield of N,N'-dimethyl-1,2-ethanediamine was less than 70 % so that it was not satisfactory industrially.

## SUMMARY OF THE INVENTION

The inventors have studied to improve the process for production of N,N'-dialkylalkanediamines by condensation reaction of dichloroalkanes with lower alkyl primary amines and as a result, have found that water entrained in the reaction system or vessel has, surprisingly, a large influence on the yield of end product.

In accordance with this invention, there is provided a process for producing of N,N'-dialkylalkanediamines represented by the formulae (I), (II) or (III),

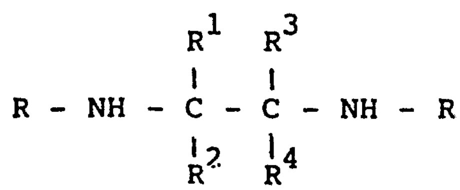
(I)

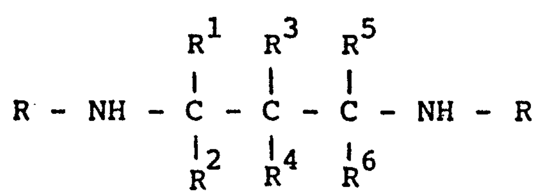
(II)

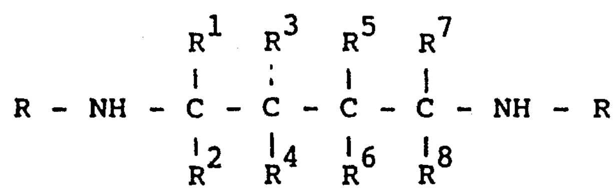
(III)

wherein R is a lower alkyl group and $R^1$ to $R^8$ are each a hydrogen atom or a lower alkyl group, which comprises reacting the corresponding dichloroalkanes represented by Formulae (IV), (V) or (VI),

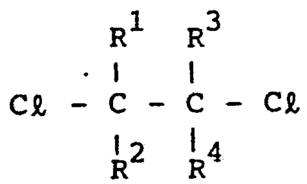

(IV)

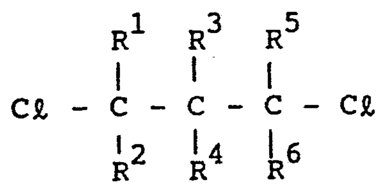

(V)

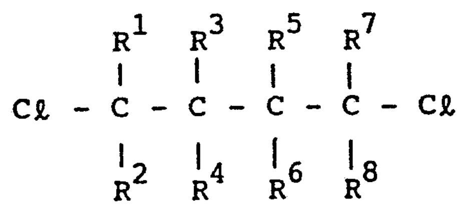

(VI)

wherein $R^1$ to $R^8$ are as defined above with a lower alkyl primary amine represented by Formula (VII),

$R-NH_2$ (VII)

wherein R is as defined above substantially in the absence of water.

DETAILED DESCRIPTION OF THE INVENTION

Examples of dichloroalkanes of the formulae (VI), (V) or (VI) include 1,2-dichloroethane, 1,2-dichloropropane, 1,3-dichloropropane, 1,3-dichloro-2,2-dimethylpropane, 1,3-dichlorobutane and 1,4-dichlorobutane.

Examples of the lower alkyl primary amine of the formula (VII) include methylamine, ethylamine, 1-methylethylamine and propylamine.

In the process of this invention, dichloroalkanes and lower alkyl primary amines should be used in the form of not substantially containing water. As for alkylamines of low boiling point such as methylamine and ethylamine, a liquefied anhydrous one is easily available from a bomb which is filled with it under elevated pressure.

Examples of the corresponding N,N'-dialkylalkanediamines of the formulae (I), (II) or (III) include N,N'-dimethyl-1,2-ethanediamine, N,N'-dimethyl-1,2-propanediamine, N,N'-dimethyl-1,3-propanediamine, N,N',2,2-tetramethyl-1,3-propanediamine, N,N'-dimethyl-1,3-butanediamine, N,N'-dimethyl-1,4-butanediamine, N,N'-diethyl-1,2-ethanediamine and N,N'-bis(1-methylethyl)-1,2-ethanediamine.

The reaction temperature is not particularly limited, though it is within the range of 0° to 200°C, preferably 50° to 150°C.

The reaction pressure may vary depending on the selected conditions, though it is within the range of normal pressure to 50 bars.G ($kg/cm^2G$), preferably 2 to 30 bars.G ($kg/cm^2G$).

A preferred embodiment of this invention is usually effected as follows:

Dichloroalkanes and lower alkyl primary amines

substantially not containing water are fed to an autoclave provided with a temperature measuring apparatus and a mechanical stirring apparatus. In case the lower alkyl primary amine can be fed under normal temperature and normal pressure, it may be fed simultaneously with addition of dichloroalkanes. When using a lower alkyl primary amine such as methylamine which is gaseous under normal pressure-normal temperature and therefore, is difficult to be fed, dichloroalkanes are charged into an autoclave which is thereafter sealed hermetically. Then, the autoclave is connected with a liquefied alkylamine bomb through a conduit, the liquefied alkylamine is fed from the bomb to the autoclave till the desired molar concentration is obtained and thereafter, reaction is completed at desired temperature. After completion of the reaction, N,N'-dialkylalkanediamines can be isolated by conventional methods such as distillation.

EFFECTS OF THE INVENTION

According to the process of this invention carrying out the reaction substantially in the absence of water, end products are obtained with a high yield and a good efficiency per reactor capacity as compared with the case of carrying out the reaction in the presence of water.

This invention will be illustrated by non-limitative examples and comparative examples.

Example 1

26.7 g (0.22 mols) of 1,2-dichloroethane are charged to a 300 mℓ autoclave made of tantalum, which is then sealed hermetically. Thereafter, the autoclave is connected with a liquefied methylamine bomb through a conduit and 100.6 g (2.64 mols) of methylamine are fed. The internal temperature of the autoclave is elevated and reaction is effected at 100°C for two hours. After completion of the reaction the reaction product mixture is analysed by gas chromatography.

Yield of N,N'-dimethyl-1,2-ethanediamine: 77.3 %

Comparative Example 1

15.8 g (0.16 mols) of 1,2-dichloroethane and 149.2 g of a 40 % aqueous solution of methylamine (1.92 mols of methylamine) are charged to a 300 mℓ autoclave made of tantalum, which is then sealed hermetically. The internal temperature of the autoclave is elevated and reaction is carried out at 100°C for two hours. Thereafter the reaction product mixture is analysed with gas chromatography.

Yield of N,N'-dimethyl-1,2-ethanediamine: 69.6 %

Examples 2-6; Comparative Examples 2-6

The reaction is carried out in the same manner as in Example 1 and Comparative Example 1 varying the type and amount of dichloroalkanes and alkylamines, respectively as set forth in Table 1.

The reaction product mixture is analysed by gas chromatography to determine the yield. The results obtained are set forth in the Table 1.

Table 1

| Example No. | Dichloroalkanes Type | Amount g | Amount mol | Alkylamines Type | Amount g | Amount mol | Amine/ Dichloro mol ratio | Reaction Conditions Temp. (°C) | Time (hrs) | End Product Type | Yield |
|---|---|---|---|---|---|---|---|---|---|---|---|
| Ex. 1 | 1,2-Dichloroethane | 26.7 | 0.27 | Pure methylamine | 100.6 | 3.24 | 12 | 100 | 2 | N,N'-dimethyl-1,2-ethanediamine | 77.7 |
| Comp. Ex. 1 | " | 15.8 | 0.16 | 40% methylamine aq. sol. | 149.2 | 1.92 | " | " | " | " | 69.6 |
| Ex. 2 | 1,2-Dichloropropane | 30.5 | 0.27 | Pure methylamine | 100.6 | 3.24 | " | " | " | N,N'-dimethyl-1,2-propanediamine | 79.5 |
| Compar. Ex. 2 | " | 18.1 | 0.16 | 40% methylamine aq. sol. | 149.2 | 1.92 | " | " | " | " | 72.3 |
| Ex. 3 | 1,3-Dichloropropane | 30.5 | 0.27 | Pure methylamine | 100.6 | 3.24 | " | " | " | N,N'-dimethyl-1,3-propanediamine | 77.9 |
| Compar. Ex. 3 | " | 18.1 | 0.16 | 40% methylamine aq. sol. | 149.2 | 1.92 | " | " | " | " | 70.1 |
| Ex. 4 | 1,4-Dichlorobutane | 34.3 | 0.27 | Pure methylamine | 100.6 | 3.24 | " | " | " | N,N'-dimethyl-1,4-butanediamine | 78.7 |
| Compar. Ex. 4 | " | 20.3 | 0.16 | 40% methylamine aq. sol. | 149.2 | 1.92 | " | " | " | " | 73.2 |
| Ex. 5 | 1,2-Dichloroethane | 26.7 | 0.27 | Pure ethylamine | 146.1 | 3.24 | " | " | " | N,N'-diethyl-1,2-ethanediamine | 79.1 |
| Compar. Ex. 5 | " | 15.8 | 0.16 | 40% ethylamine aq. sol. | 216.5 | 1.92 | " | " | " | " | 72.4 |

CLAIMS:

1. A process for producing of N,N'-dialkylalkanediamines represented by the formulae (I), (II) or (III),

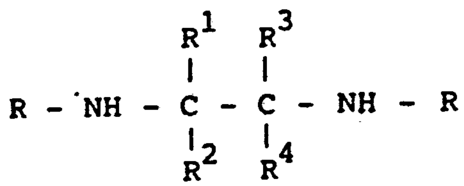

(I)

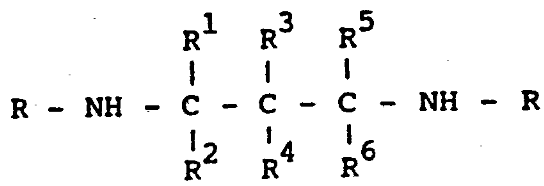

(II)

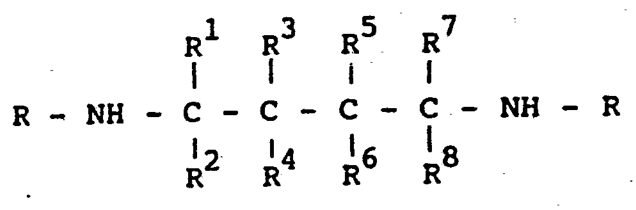

(III)

wherein R is a lower alkyl group and $R^1$ to $R^8$ are each hydrogen atom or a lower alkyl group, which comprises reacting the corresponding dichloroalkanes represented by Formulae (IV), (V) or (VI),

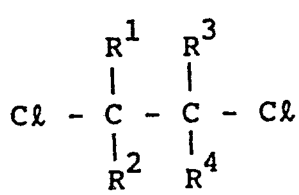

(VI)

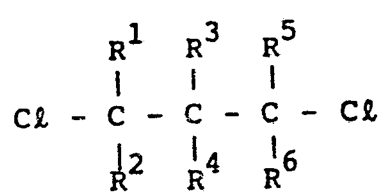

(V)

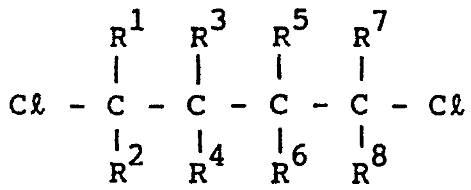

(VI)

wherein $R^1$ to $R^8$ are as defined above with a lower alkyl primary amine represented by Formula (VII),

$$R\text{-}NH_2 \qquad \text{(VII)}$$

wherein R is as defined above, substantially in the absence of water.

2. The process of claim 1 wherein said lower alkyl primary amine is a liquefied anhydrous compound having a low boiling point.

3. The process of claim 1 wherein said lower alkyl primary amine is liquefied anhydrous methylamine or ethylamine.

0254229

European Patent Office

# EUROPEAN SEARCH REPORT

Application number

EP 87 11 0406

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | US-A-2 334 782 (E.L. MARTIN)<br>* Claims; examples * | 1 | C 07 C 85/04<br>C 07 C 87/14 |
| Y | GB-A-1 469 615 (BAYER)<br>* Claims * | | |
| Y | GB-A-2 013 180 (AMERICAN CYANAMID)<br>* Claims * | 1 | |
| Y | BE-A- 545 722 (BASF)<br>* Claims; examples * | 1 | |

| TECHNICAL FIELDS SEARCHED (Int. Cl 4) |
|---|
| C 07 C 85/00<br>C 07 C 87/00 |

The present search report has been drawn up for all claims

| Place of search | Date of completion of the search | Examiner |
|---|---|---|
| THE HAGUE | 26-10-1987 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document